# EUROPEAN PATENT SPECIFICATION

(11) **EP 3 338 802 B1**
(45) Date of publication and mention of the grant of the patent: **20.02.2019**
(21) Application number: 16425117.5
(22) Date of filing: 22.12.2016
(51) Int. Cl.: A61K 47/02, A61K 9/16, A61K 31/198

(54) **BIPHASIC CREATINE NUTRACEUTIC COMPOSITION**
ZWEIPHASIGE CREATINE NUTRACEUTISCHE ZUSAMMENSETZUNG
COMPOSITION NEUTRACEUTIQUE BIPHASÉE CONTENANT DE CREATINE

(43) Date of publication of application: 27.06.2018
(73) Proprietor: EKALAB S.R.L., 31050 Ponzano Veneto (TV) (IT)
(72) Inventor: SPOGLI, Roberto, 06126 Perugia (IT); SISANI, Michele, 06125 Perugia (IT); SALVADORI Giuseppe, 31030 Casier Loc. Dosson (IT); PERIOLI, Luana, 06012 Citta'di Castello (IT); PAGANO, Cinzia, 06127 Perugia (IT)
(74) Representative: Baldi, Claudio

(56) References cited:
- EP-A1- 1 859 794
- WO-A1-2005/099455
- WO-A1-2006/066637
- WO-A2-2012/034201
- GB-A- 2 429 915

## Description

The present patent application relates to a biphasic creatine nutraceutic composition.

Creatine, or methylguanidinoacetic acid, is an endogenous product of metabolism in mammals, and is bio-synthesized starting from arginine, S-adenosylmethionine and glycine. 95% of creatine in the human body is localized in skeletal muscles, and is used to regenerate ATP during the first seconds of muscular contraction.

However, during an intense physical training session, the ATP in muscular fibers is consumed very rapidly and therefore its concentration is extremely low. It is therefore necessary to restore ATP by taking creatine.

Creatine intake regimes are known, which provide for a dosage of max. 20/g per day. However, such a contribution is much higher than the creatine captation ability of skeletal muscles, determining an excessive creatine concentration at plasmatic level with consequent disorders, such as formation of creatinine, renal fatigue and formation of molecules that are harmful for the body.

Moreover, the administration of high amounts of creatine in the gastrointestinal system results in an increase of creatine plasmatic concentrations, without increasing the captation of creatine of muscles.

By administering creatine-based formulations with a single dissolution rate (either high or low), fluctuations of creatine plasmatic concentrations are obtained due to the fact that the elimination mechanisms implemented by the body proceed rapidly between two successive administrations, thus causing a drop of creatine plasmatic concentrations. The patent GB 2429915 discloses a sustained release formulation containing 50-70% of creatine and as excipient 0.5-1,5% Mg stearate. Possible use of bilayer tableting technology that may be used in the practice of the invention. DUREDAS was developed to provide for two different delivery rates, or dual delivery of a drug from one dosage form. the "controlled delivery" for the purposes of the present invention can include 1) the delivery of the creatine bolus to the gastro-intestinal tract; 2) the delivery of creatine at specific rates, as to provide a specific plasma creatine concentration range; and 3) the delivery of creatine to maximise the duration that plasma creatine is maintained within a specific concentration range. No specific biphasic example and no Mg(OH)2 is present.

Moreover, the intake of a single form of creatine, either in the form of accelerated or slowed dissolution, generates discontinuous plasmatic concentrations and exposes the body to the risk of being in an overdose condition, with the elimination of the non-absorbed creatine, and/or the formation of creatinine (which is typical of fast-release formulations); on the contrary, another risk for the body is the lack of creatine, with a negative deviation from the effective concentration (which is typical of slow release formulations).

The main purpose of the present invention is to provide a creatine-based composition that is able to optimize the absorption of creatine and reduce urinary secretion, formation of creatinine, metabolic fatigue and formation of metabolism by-products.

Another purpose of the present invention is to provide a creatine-based composition that is able to achieve and maintain an effective blood creatine concentration for the absorbed creatine to be captured and used by the skeletal muscle.

These purposes are achieved according to the present invention with the characteristics described in the independent claim 1.

Advantageous embodiments of the invention will appear from the dependent claims.

The biphasic creatine nutraceutic composition of the invention has a total creatine content in the range between 50% and 90% by weight, preferably 73% by weight, and comprises one creatine fast-release component and one creatine slow-release component.

Moreover such a composition advantageously comprises excipients, such as microcrystalline cellulose, hydroxy-propyl-cellulose, mono- and diglycerides of fatty acids and/or silicon dioxide.

The applicant made several experimental tests and observed that the intake of such a composition favors blood creatine concentrations in the range of 0.30 mmol/L and 0.70 mmol/L for 5-6 hours after taking the dose.

In fact, the fast-release composition immediately increases the plasmatic creatine concentration to values in the range between 0.30 mmol/L and 0.70 mmol/L and keeps the plasmatic concentration in the range between 0.30 mmol/L and 0.70 mmol/L for approximately three hours. Instead, the slow-release component starts giving a significant contribution of creatine between the third and the sixth hour after taking the dose, keeping the plasmatic concentration in the range between 0.30 mmol/L and 0.70 mmol/L for 5-6 hours after the intake.

In particular, the fast-release component has a creatine release percentage in the range between 70% and 100% within 50 minutes. Instead, the slow-release component has a creatine release percentage in the range between 40% and 80% within 120 minutes after taking the dose, and in the range between 60% and 100 % within 300 minutes after taking the dose.

The creatine release speed of the fast-release component and of the slow-release component was measured with a dissolution test in compliance with European Pharmacopoeia 8.0. In particular, each dissolution test was carried out by providing samples containing 140 mg of creatine monohydrate and positioning said samples in intestinal fluid at pH 7.4 (5 ml) thermostated at 37.0°C ± 0.1. The samples were kept under agitation in a thermostated shaker (50-100 rpm) to simulate intestinal movements. The use of a limited fluid volume permitted to work with high creatine concentrations in a way to assess the behavior in oversaturation (double solubility) conditions, simulating at best what may occur in the intestinal lumen where creatine is in a limited fluid volume. In set periods of time, i.e. 15', 30', 1h, 2h, 3h and 5h from the beginning of the test, aliquots of each sample (0.5 ml) were taken and the volume was restored every time with phosphate buffer at 7.4 pH at 37.0 °C in such a way to have a constant final volume. The creatine concentration was determined in each sample via HPLC (High Performance Liquid Chromatography) and the release percentage was calculated from the creatine concentration.

The slow-release component is realized with a carrying process of creatine monohydrate on magnesium hydroxide such to modulate the release speed of creatine in the intestinal environment. In particular, by making several comparative dissolution tests only with creatine monohydrate and with creatine supported on magnesium hydroxide, the applicant surprisingly discovered that the creatine supported on magnesium hydroxide has a slowed dissolution profile compared to creatine monohydrate. In fact, by using suitable ratios between hydroxide magnesium and creatine, the applicant obtained mixtures in which the creatine dissolution speed was modulated according to the ratio between the two components, the collision energies between particles and the treatment time. Such a result can be explained with the fact that stable weak interactions, typically hydrogen bonds, are established between the magnesium hydroxide and creatine, which modify the dissolution rate of creatine.

In particular, the slow-release component comprises a mixture of magnesium hydroxide and creatine monohydrate, wherein the percentage of magnesium hydroxide is in the range between 20% and 40%, preferably 30% by weight.

The composition comprises a percentage by weight of the slow-release component in the range between 30% and 50% by weight, preferably 41% by weight. The slow-release component has a final particle size in the range between 1 µm and 150 µm with an average particle size D₅₀ in the range between 10 µm and 50 µm, preferably between 15 µm and 30 µm.

The preparation method of the slow-release component of the biphasic nutraceutic composition according to the invention comprises the following steps:
- mixing a composition of magnesium hydroxide and creatine monohydrate in a mixer for 20-40 minutes in a way to obtain a magnesium-hydroxide-creatine monohydrate mixture;
- micronization of the magnesium hydroxide-creatine monohydrate mixture in a centrifugal micronizer with ventilated grinding at a rotational speed of 19-20 m/s and for a period of time of 15-25 minutes;
- stabilization of the micronized mix for a time interval in the range between 3 and 15 minutes in a way to obtain a stabilized mixture;
- final micronization of the magnesium hydroxide-creatine monohydrate mixture in a centrifugal micronizer with ventilated grinding at a rotational speed of 15-25 rpm for 15-25 minutes;
- sieving of the mixture with a 90-110 micron selector filter.

Advantageously, such a method provides for using a composition comprising 900g of magnesium hydroxide and 2,100 g of monohydrate creatine.

The fast-release component comprises creatine monohydrate and, advantageously, creatine citrate, creatine piruvate, creatine ester, and/or other types of creatine in salt, kelate or conjugate form.

In particular, the fast-release component comprises creatine monohydrate with a particle size with D₉₀<400 micron, D₅₀<180 micron and D₁₀<56 micron, preferably D₉₀<250 micron, D₅₀<80 micron and D₁₀<30 micron.

The composition is made as a tablet coated by a gastroresistant film with intestinal dissolution that belongs to the following chemical classes:
- acrylic polymers and acrylic and methacrylic copolymers (Eudragit S and Eudragit L);
- cellulose derivatives: cellulose acetate phthalate (CAP) as Aquateric pseudolatex in aqueous version - cellulose acetate trimellitate (CAT), hydroxypropylmethylcellulose acetate succinate (HPMCAS) and hydroxypropylmethylcellulose phthalate (HP-F);
- Vinyl polymers: polyvinyl acetate phthalate (PVAP) as such, or in latex version (Sureteric).

The tablet has a mass in the range from 0.50 g and 3.00 g, preferably between 1.00 g and 2.00 g. The gastroresistant film of the tablet has a mass in the range between 1% and 15% by weight with respect to the mass of the tablet, preferably in the range between 3% and 8%.

Advantageously, such a tablet is administered according to a posologic regime that provides for taking 4 doses per day for 3 to 10 days, in which each dose is composed of two tablets of approximately 1.5 g each. The use of a biphasic composition, that is a combination of fast-release creatine, as fast-release dose, and slow-release creatine, composed of creatine supported on magnesium hydroxide, permits a bimodal creatine release, achieving and maintaining an effective plasmatic concentration.

The advantages of the composition according to the present invention versus the compositions of the prior art, which only comprise fast-release creatine monohydrate, are evident.

## Claims

1. Biphasic creatine nutraceutic composition, comprising:
- a creatine fast-release component, and
- a creatine slow-release component comprising creatine supported on magnesium hydroxide.

2. The nutraceutic composition of claim 1, wherein the percentage by weight of the slow-release component is in the range between 50% and 30% in weight, preferably 41% in weight.

3. The nutraceutic composition of claim 1 or 2, wherein the slow-release component comprises a mixture of magnesium hydroxide and creatine monohydrate, wherein the percentage by weight of magnesium hydroxide is in the range between 20% and 40%, preferably 30% by weight.

4. The nutraceutic composition of any one of the preceding claims, wherein the slow-release component has a final particle size in the range between 1 µm and 150 µm with an average particle dimension D₅₀ in the range between 10 µm and 50 µm, preferably between 15 µm and 30 µm.

5. The nutraceutic composition of any one of the preceding claims, wherein the slow-release component has a creatine release percentage in the range between 40% and 80% within 120 minutes, and in the range between 60% and 100% within 300 minutes.

6. The nutraceutic composition of any one of the preceding claims, wherein the composition also comprises excipients, such as microcrystalline cellulose, hydroxy-propyl-cellulose, mono- and diglycerides of fatty acids and/or silicon dioxide.

7. The nutraceutic composition of any one of the preceding claims, wherein the composition has a total creatine content in the range between 50% and 90% by weight, preferably 73% by weight.

8. The nutraceutic composition of any one of the preceding claims, wherein the creatine fast-release component comprises creatine monohydrate.

9. The nutraceutic composition of clam 8, wherein the fast-release component also comprises creatine citrate, creatine piruvate, ester creatine.

10. The nutraceutic composition of any one of the preceding claims, wherein the fast-release component has a creatine release percentage in the range between 70% and 100% within 50 minutes.

11. The nutraceutic composition of any one of the preceding claims, wherein the fast-release component comprises creatine monohydrate with a particle size with D₉₀<400 micron, D₅₀<180 micron and D₁₀<56 micron, preferably D₉₀<250 micron, D₅₀<80 micron and D₁₀<30 micron.

12. Tablet comprising the nutraceutic composition of biphasic type according to any one of claims 1 to 11; said tablet being coated by a gastroresistant film with intestinal dissolution that belongs to the following chemical classes:
- Acrylic polymers and acrylic and methacrylic copolymers;
- Cellulose derivatives: cellulose acetate phthalate as Aquateric pseudolatex in aqueous version, cellulose acetate trimellitate, hydroxypropylmethylcellulose acetate succinate and hydroxypropylmethylcellulose phthalate;
- Vinyl polymers: polyvinyl acetate phthalate as such, or in latex version.

13. The tablet of claim 12, wherein said tablet has a mass in the range between 0.50 g and 3.00 g, preferably in the range between 1.00 g and 2.00 g.

14. The tablet of claim 12 or 13, wherein the gastroresistant film of the tablet has a mass in the range between 1% and 15% by weight with respect to the mass of the tablet, preferably in the range between 3% and 8%.

15. Preparation method of the slow-release component of the biphasic nutraceutic composition of any one of claims 1 to 11, said method comprising the following steps:
- mixing a composition of magnesium hydroxide and creatine monohydrate in a mixer for 20-40 minutes in a way to obtain a magnesium-hydroxide-creatine monohydrate mixture;
- micronization of the magnesium hydroxide-creatine monohydrate mixture in a centrifugal micronizer with ventilated grinding at rotational speed of 19-20 m/s and for a period of time of 15-25 minutes;
- stabilization of the micronized mix for a time interval in the range between 3 and 15 minutes in a way to obtain a stabilized mixture;
- final micronization of the magnesium hydroxide-creatine monohydrate mixture in a centrifugal micronizer with ventilated grinding at a rotational speed of 15-25 rpm for 15-25 minutes;
- sieving the mixture with a 90-110 micron selector filter.

## Patentansprüche

1. Biphasische nutrazeutische, kreatinhaltige Zusammensetzung, umfassend:
- einen Bestandteil mit schneller Freisetzung von Kreatin und
- einen Bestandteil mit langsamer Freisetzung von Kreatin, umfassend Kreatin auf Magnesiumhydroxid als Träger.

2. Nutrazeutische Zusammensetzung nach Anspruch 1, wobei der Gewichtsprozentsatz des langsam freigesetzten Bestandteils im Bereich von 50 bis 30 Gew.-%, vorzugsweise bei 41 Gew.-% liegt.

3. Nutrazeutische Zusammensetzung nach Anspruch 1 oder 2, wobei der langsam freigesetzte Bestandteil eine Mischung aus Magnesiumhydroxid und Kreatinmonohydrat umfasst, wobei der Gewichtsprozentsatz von Magnesiumhydroxid im Bereich von 20 bis 40 Gew.-%, vorzugsweise bei 30 Gew.-% liegt.

4. Nutrazeutische Zusammensetzung nach einem der vorstehenden Ansprüche, wobei der langsam freigesetzte Bestandteil eine finale Partikelgröße im Bereich von 1 bis 150 µm mit einem durchschnittlichen Partikeldurchmesser D₅₀ im Bereich von 10 bis 50 µm, vorzugsweise 15 bis 30 µm aufweist.

5. Nutrazeutische Zusammensetzung nach einem der vorstehenden Ansprüche, wobei der langsam freigesetzte Bestandteil einen Prozentsatz der Kreatinfreisetzung im Bereich von 40 % bis 80 % innerhalb von 120 Minuten und im Bereich von 60 % bis 100 % innerhalb von 300 Minuten aufweist.

6. Nutrazeutische Zusammensetzung nach einem der vorstehenden Ansprüche, wobei die Zusammensetzung ferner Hilfsstoffe wie mikrokristalline Cellulose, Hydroxypropylcellulose, Mono- und Diglyceride von Fettsäuren und/oder Siliciumdioxid umfasst.

7. Nutrazeutische Zusammensetzung nach einem der vorstehenden Ansprüche, wobei die Zusammensetzung einen Gesamtgehalt an Kreatin um Bereich von 50 bis 90 Gew.-%, vorzugsweise von 73 Gew.-% aufweist.

8. Nutrazeutische Zusammensetzung nach einem der vorstehenden Ansprüche, wobei der schnell freigesetzte Bestandteil Kreatinmonohydrat umfasst.

9. Nutrazeutische Zusammensetzung nach Anspruch 8, wobei der schnell freigesetzte Bestandteil ferner Kreatincitrat, Kreatinpyruvat und Kreatinester umfasst.

10. Nutrazeutische Zusammensetzung nach einem der vorstehenden Ansprüche, wobei der schnell freigesetzte Bestandteil einen Prozentsatz der Kreatinfreisetzung im Bereich von 70 % bis 100 % innerhalb von 50 Minuten umfasst.

11. Nutrazeutische Zusammensetzung nach einem der vorstehenden Ansprüche, wobei der schnell freigesetzte Bestandteil Kreatinmonohydrat mit einer Partikelgröße D₉₀<400 Mikrometer, D₅₀<180 Mikrometer und D₁₀<56 Mikrometer, vorzugsweise D₉₀<250 Mikrometer, D₅₀<80 Mikrometer und D₁₀<30 Mikrometer umfasst.

12. Tablette umfassend die nutrazeutische Zusammensetzung vom biphasischen Typ nach einem der Ansprüche 1 bis 11; wobei die Tablette mit einem magensaftresistenten, im Darm auflösbaren Überzug versehen ist, der folgenden chemischen Klassen angehört:
- Acrylpolymere und Acryl- und Methacryl-Copolymere;
- Cellulosederivate: Celluloseacetatphthalat wie Aquateric Pseudolatex in wässriger Version - Celluloseacetatrimellitat, Hydroxypropylmethylcelluloseacetat-Succinat und Hydroxypropylmethylcellulosephthalat,
- Vinylpolymere: Polyvinylacetatphthalat in unverändertem Zustand oder in Latexform.

13. Tablette nach Anspruch 12, wobei die Tablette eine Masse im Bereich von 0,50 bis 3,00 g, vorzugsweise im Bereich von 1,00 bis 2,00 g aufweist.

14. Tablette nach Anspruch 12 oder 13, wobei der magensaftresistente Überzug der Tablette eine Masse im Bereich von 1 bis 15 Gew.-% in Bezug auf die Masse der Tablette, vorzugsweise im Bereich 3 bis 8 Gew.-% aufweist.

15. Verfahren zur Herstellung des schnell freigesetzten Bestandteils der biphasischen, nutrazeutischen Zusammensetzung nach einem der Ansprüche 1 bis 11, wobei das Verfahren die folgenden Schritte umfasst:
- Mischen einer Zusammensetzung aus Magnesiumhydroxid und Kreatinmonohydat in einem Mischer für 20-40 Minuten, um eine Mischung aus Magnesiumhydroxid und Kreatinmonohydat zu erhalten;
- Mikronisieren der Mischung aus Magnesiumhydroxid und Kreatinmonohydat in einer zentrifugalen Strahlmühle mit ventiliertem Mahlen bei einer Rotationsgeschwindigkeit von 19-20 m/s für einen Zeitraum von 15-25 Minuten;
- Stabilisieren der mikronisierten Mischung für einen Zeitintervall im Bereich von 3 bis 15 Minuten, um eine stabilisierte Mischung zu erhalten;
- finales Mikronisieren der Mischung aus Magnesiumhydroxid und Kreatinmonohydat in einer zentrifugalen Strahlmühle mit ventiliertem Mahlen bei einer Rotationsgeschwindigkeit von 15-25 U/min für einen Zeitraum von 15-25 Minuten;
- Abfiltrieren der Mischung in einem Selektorfilter zu 90-110 Mikrometer.

## Revendications

1. Composition nutraceutique de créatine du type biphasique comprenant :
- un composant à libération rapide de créatine, et
- un composant à libération contrôlée de créatine comprenant une créatine supportée sur hydroxyde de magnésium.

2. Composition nutraceutique selon la revendication 1, où le pourcentage en poids du composant à libération contrôlée est compris dans la plage 50-30% en poids, préférablement le 41% en poids.

3. Composition nutraceutique selon la revendication 1 ou 2, où le composant à libération contrôlée comprend un mélange de magnésium hydroxyde et créatine monohydrate, où le pourcentage en poids de magnésium hydroxyde est compris dans la plage 20-40%, préférablement égal à 30% en poids.

4. Composition nutraceutique selon l'une quelconque des revendications précédentes, où le composant à libération contrôlée a une granulométrie finale comprise entre 1-150 µm avec une dimension moyenne des particules D₅₀ comprise dans la plage 10-50 µm, préférablement entre 15-30 µm.

5. Composition nutraceutique selon l'une quelconque des revendications précédentes, où le composant à libération contrôlée présente un pourcentage de libération de créatine compris entre 40% et 80% dans le délai de 120 minutes, et compris entre 60% et 100% dans le délai de 300 minutes.

6. Composition nutraceutique selon l'une quelconque des revendications précédentes, où la composition comprend également des agents de charge tels que la cellulose microcristalline, l'hydroxypropylcellulose, les mono et diglycérides d'acides gras et/ou le dioxyde de silicium.

7. Composition nutraceutique selon l'une quelconque des revendications précédentes, où la composition a un contenu total de créatine compris dans la plage 50-90% en poids, préférablement le 73% en poids.

8. Composition nutraceutique selon l'une quelconque des revendications précédentes, où le composant à libération rapide de créatine comprend de la créatine monohydrate.

9. Composition nutraceutique selon la revendication 8, où le composant comprend également de la créatine citrate, de la créatine piruvate, de la créatine ester.

10. Composition nutraceutique selon l'une quelconque des revendications précédentes, où le composant à libération rapide présente un pourcentage de libération de la créatine compris entre 70% et 100% dans le délai de 50 minutes.

11. Composition nutraceutique selon l'une quelconque des revendications précédentes, où le composant à libération rapide comprend de la créatine monohydrate avec une granulométrie ayant un D₉₀<400 microns, D₅₀<180 microns et Dio<56 microns, préférablement D₉₀<250 microns, D₅₀<80 microns et Dio<30 microns.

12. Comprimé comprenant la composition nutraceutique de type biphasique selon l'une quelconque des revendications de 1 à 11 ; ledit comprimé étant enrobé d'une pellicule gastro-résistante à dissolution intestinale appartenant aux classes chimiques suivantes :
- Polymères acryliques et copolymères acryliques et méthacryliques ;
- Dérivés de la cellulose : Acéto-phatalate de cellulose tel que Aquateric pseudo-latex en version aqueuse - acéto-trimellitate de cellulose, acéto-succinate d'hydroxypropylméthylcellulose et phtalate d'hydroxypropylméthylcellulose.
- Polymères vinyliques : acéto-phtalate de polyvinyle (tel quel ou en version latex.

13. Comprimé selon la revendication 12, où ledit comprimé a une masse comprise entre 0,50-3,00 g, préférablement comprise entre 1,00-2,00 g.

14. Comprimé selon la revendication 12 ou 13, où la pellicule gastro-résistante du comprimé a une masse comprise entre 1-15% en poids par rapport à la masse du comprimé, préférablement entre 3-8 %.

15. Méthode pour la préparation du composant à libération contrôlée de la composition nutraceutique de type biphasique selon l'une quelconque des revendications de 1 à 11, ladite méthode comprenant les étapes suivantes :
- mélange d'une composition d'hydroxyde de magnésium et de créatine monohydrate dans un mélangeur pendant 20-40 minutes, de manière à obtenir un mélange d'hydroxyde de magnésium-créatine monohydrate ;
- micronisation du mélange d'hydroxyde de magnésium et de créatine monohydrate dans un microniseur centrifuge à moulure ventilée avec une vitesse de rotation de 19-20 m/s et pour une période de temps de 15-25 minutes ;
- stabilisation du mélange micronisé pour un intervalle de temps compris entre 3 à 15 minutes, de manière à obtenir un mélange stabilisé ;
- micronisation finale du mélange d'hydroxyde de magnésium-créatine monohydrate dans un microniseur centrifuge à moulure ventilée avec une vitesse de rotation de 15-25 tr/min pendant 15-25 minutes ;
- filtrage du mélange à l'aide d'un filtre sélecteur de 90 - 110 microns.
